# EUROPEAN PATENT SPECIFICATION

(11) **EP 2 858 566 B1**
(45) Date of publication and mention of the grant of the patent: **27.09.2017**
(21) Application number: 13719655.6
(22) Date of filing: 11.04.2013
(51) Int. Cl.: A61B 5/117

(54) **MULTI-SPECTRAL IMAGING SYSTEM AND METHOD FOR REMOTE BIOMETRIC MEASUREMENT OF HUMAN PHYSIOLOGICAL PARAMETERS**
MULTISPEKTRALES BILDGEBUNGSSYSTEM UND VERFAHREN ZUR ENTFERNTEN BIOMETRISCHEN MESSUNG VON HUMANEN PHYSIOLOGISCHEN PARAMETERN
SYSTÈME D'IMAGERIE MULTISPECTRALE ET PROCÉDÉ POUR LA MESURE BIOMÉTRIQUE À DISTANCE DE PARAMÈTRES PHYSIOLOGIQUES HUMAINS

(30) Priority: 11.06.2012 US 201213493958
(43) Date of publication of application: 15.04.2015
(73) Proprietor: Raytheon Company, Waltham, MA 0245-1449 (US)
(72) Inventor: BERTE, Marc, Ashburn, VA 20148 (US); KOGUT, John A., Bowie, Maryland 20720-4868 (US)
(74) Representative: Dentons UKMEA LLP
(86) International application number: PCT/US2013/036112
(87) International publication number: WO 2013/187999

(56) References cited:
- WO-A1-2011/042839
- WO-A1-2013/030745
- WO-A2-2013/027027
- US-A1- 2008 188 758

## Description

### TECHNICAL FIELD

Embodiments pertain to Multi-spectral Imaging (MSI) systems. Some embodiments pertain to remote biometric measurement of conditions of human subjects.

### BACKGROUND

Technologies that provide situational awareness are used in military and secured environments. Such technologies can provide information regarding the number and location of humans in a particular area. Multi-spectral Imaging (MSI) systems are technologies that can provide situational awareness.

What are needed are lower cost and effective systems that provide remote biometric measurement of conditions of human subjects. What is also needed is a multi-spectral imaging system and method for long-range detection and characterization of human subjects.

WO 2011/042839 discloses a method of characterizing periodic components of a first signal, which includes obtaining at least two signals representative of intensities of captured electromagnetic radiation, each corresponding to a respective different radiation frequency range. The first signal is derivable from an output signal obtainable by applying a transformation to the second signals such that any value of the output signal is based on values from each respective second signal at corresponding points in time.

### SUMMARY

The invention provides a method and system as claimed by independent claims 1 and 8, whereby physiological parameters of human subjects may be remotely detected using a multi-spectral imaging technique. Skin pixels are detected using a skin-detection technique and the temporal variation of the differential reflection of certain spectral signatures of the skin pixels is analyzed to determine certain human physiological parameters. The human physiological parameters include heart rate and respiration rate, and may further include blood pressure, and/or blood oxygen saturation percentage.

In accordance with some embodiments, skin pixels may be initially detected using a skin detection technique based on digital images of reflected light. Reflected light within three narrow bands is analyzed from these images to identify skin pixels. Ratios of pixel intensities in these narrow bands may be used to identify skin pixels. The three narrow bands include a 547 nm band (λ₁), a 577 nm band (λ₂) and a 607 nm band (λ₃). In some embodiments, the ratio of the sum of pixel intensities of the 547 nm band (λ₁) and the 607 nm band (λ₃) to pixel intensities of the 577 nm band (λ₂) (i.e., (λ₁ + λ₃)/ λ₂) may be used to identify skin pixels, although this is not a requirement.

Once the skin pixels are detected and identified, the temporal variation of the differential reflection of certain spectral signatures of the skin pixels may be analyzed to determine certain human physiological parameters. In these embodiments, the temporal variation of the differential reflection due to the pumping of the heart may be used to determine certain human physiological parameters. In some embodiments, the temporal variation of this relative reflectance may be analyzed to heart rate, respiration rate, blood pressure and/or blood oxygen saturation percentage. In some embodiments, differential reflectance may be analyzed at one or more wavelengths that provide a difference signature arising from the relative presence of oxygenated hemoglobin and de-oxygenated hemoglobin underlying the skin being observed. In some embodiments, the differential reflectance may be analyzed based on wavelengths in the 650 nm band (λ₄) and/or wavelengths in the 780 nm band (λ₅). These embodiments are described in more detail below. The 650 nm band may include wavelengths at 650 nm +/- 20 nm and the 780 nm band may include wavelengths at 780 nm +/- 20 nm. In some embodiments, the 650 nm band may include wavelengths at 650 nm +/- 30 nm and the 780 nm band may include wavelengths at 780 nm +/- 30 nm.

The temporal variation of the ratio of pixel intensities of light reflected from a subject in the 650 nm band (λ₄) divided by a pixel intensity of light reflected from the subject 577 nm band (λ₂) is analyzed to determine certain human physiological parameters. The ratio of the pixel intensity of the light reflected from the subject in the 650 nm band (λ₄) divided by a pixel intensity of the light reflected from the subject in the 780 nm band (λ₅) is also analyzed to determine certain human physiological parameters, as described in more detail below. The frequency content of the temporal variation of the ratios of these pixel intensities is also analyzed to determine certain human physiological parameters, as described in more detail below.

Through the detection and analysis of these narrow bands, embodiments of the present invention disclosed herein may allow for long-range detection and characterization of human subjects.

### BRIEF DESCRIPTION OF THE DRAWINGS

FIG. 1 is a block diagram of a multi-spectral imaging system that provides remote biometric measurements in accordance with some embodiments;
FIG. 2 is a block diagram of a multi-spectral imaging system that provides remote biometric measurements in accordance with some embodiments;
FIG. 3 is a flowchart illustrating an example method of multi-spectral imaging in accordance with some embodiments;
FIG. 4 is a flowchart illustrating an example method of multi-spectral imaging in accordance with some embodiments;
FIG. 5 is a plot of a variation of a first ratio and a second ratio in accordance with some embodiments.
FIG. 6 is a block diagram of a computer processor system in connection with which one or more embodiments of the present disclosure can operate; and FIG. 7 is a block diagram of an integrated circuit chip in accordance with some embodiments.

### DETAILED DESCRIPTION

The following description and the drawings sufficiently illustrate specific embodiments to enable those skilled in the art to practice them. Other embodiments may incorporate structural, logical, electrical, process, and other changes. Portions and features of some embodiments may be included in, or substituted for, those of other embodiments. Embodiments set forth in the claims encompass all available equivalents of those claims.

The inventors have discovered that the challenge of remote biometric measurement of conditions of human subjects, as well as others, may be addressed by collecting light reflected from a subject and analyzing the light to monitor time-varying physiological parameters of the subject.

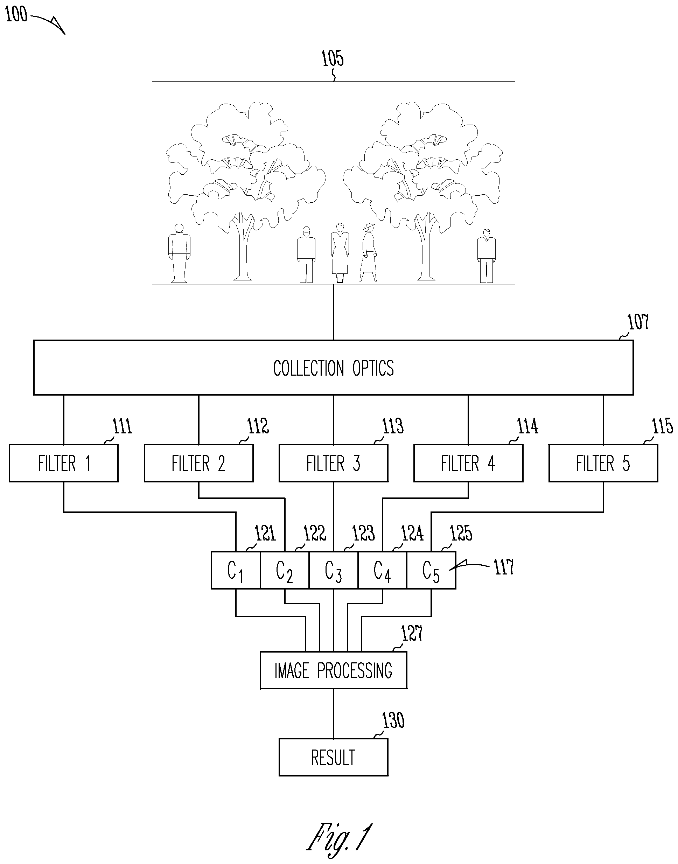

FIG. 1 is a block diagram of a multi-spectral imaging system 100 that provides remote biometric measurements in accordance with some embodiments. Light reflecting from a subject or a number of subjects is captured in a time series of images 105 received by a collection optics system 107. The light can include visible light or infrared light or both visible light and infrared light. The subjects in the time series of images 105 can include one or more humans. The collection optics system 107 splits and directs the light through five narrow band filters 111, 112, 113, 114 and 115. The filters 111-115 may each be centered about a wavelength to filter light in a band around the wavelength. The filters 111-115 can each be centered about a first wavelength, while also being separated one from another by a wavelength interval.. For example, the filters 111-115 may be centered about 577 nm +/- 20-40 nm and separated by approximately 30-50 nm. In addition, each filter 111-115 has a width of approximately 5-10 nm in accordance with some embodiments. The filter 111 may be centered about a wavelength of 547 nanometers. The filter 112 may be centered about a wavelength of 577 nanometers. The filter 113 may be centered about a wavelength of 607 nanometers. The filter 114 may be centered about a wavelength of 650 nanometers. The filter 115 may be centered about a wavelength of 780 nanometers. The multi-spectral imaging system 100 may include more or fewer than five filters in accordance with some embodiments.

The light passing through the filters 111-115 may be projected onto an image capture system 117 having a plurality of image capture zones 121, 122, 123, 124 and 125. The image capture zones 121-125 may be Multi-spectral Imaging (MSI) sensors. The image capture zones 121-125 can transform the light from the filters 111-115 into a corresponding plurality of digital images provided to an image and signal processing system 127. The image and signal processing system 127 implements algorithms to produce results 130 including temporal signatures of physiological parameters of subjects present in the time series of images 105. The temporal signatures of physiological parameters may be indicative of a human subject or subjects in the time series of images 105.

In accordance with some embodiments, multi-spectral imaging system 100 may initially detect skin pixels using a skin detection technique based on digital images of reflected light. Reflected light within three narrow bands may be analyzed from these images to identify skin pixels. Ratios of pixel intensities in these narrow bands may be used to identify skin pixels. In some embodiments, the three narrow bands may include a 547 nm band (λ₁), a 577 nm band (λ₂) and a 607 nm band (λ₃). In some embodiments, the ratio of the sum of pixel intensities of the 547 nm band (λ₁) and the 607 nm band (λ₃) to pixel intensities of the 577 nm band (λ₂) (i.e., (λ₁ + λ₃)/ λ₂) may be used to identify skin pixels, although this is not a requirement.

Once the skin pixels are detected and identified, the temporal variation of the differential reflection of certain spectral signatures of the skin pixels may be analyzed to determine certain human physiological parameters. In these embodiments, the temporal variation of the differential reflection due to the pumping of the heart may be used to determine certain human physiological parameters. In some embodiments, the temporal variation of this relative reflectance may be analyzed to heart rate, respiration rate, blood pressure and/or blood oxygen saturation percentage. In some embodiments, differential reflectance may be analyzed at one or more wavelengths that provide a difference signature arising from the relative presence of oxygenated hemoglobin and de-oxygenated hemoglobin underlying the skin being observed (e.g., the between oxygen-rich blood and oxygen-poor blood). In some embodiments, the differential reflectance may be analyzed based on wavelengths in the 650 nm band (λ₄) and/or wavelengths in the 780 nm band (λ₅). These embodiments are described in more detail below. The 650 nm band may include wavelengths at 650 nm +/- 20 nm and the 780 nm band may include wavelengths at 780 nm +/- 20 nm. In some embodiments, the 650 nm band may include wavelengths at 650 nm +/- 30 nm and the 780 nm band may include wavelengths at 780 nm +/- 30 nm.

In some embodiments, the temporal variation of the ratio of pixel intensities of light reflected from a subject in the 650 nm band (λ₄) divided by a pixel intensity of light reflected from the subject 577 nm band (λ₂) may be analyzed to determine certain human physiological parameters. The ratio of the pixel intensity of the light reflected from the subject in the 650 nm band (λ₄) divided by a pixel intensity of the light reflected from the subject in the 780 nm band (λ₅) may also be analyzed to determine certain human physiological parameters. These embodiments are described in more detail below. The frequency content of the temporal variation of the ratios of these pixel intensities may also be analyzed to determine certain human physiological parameters.

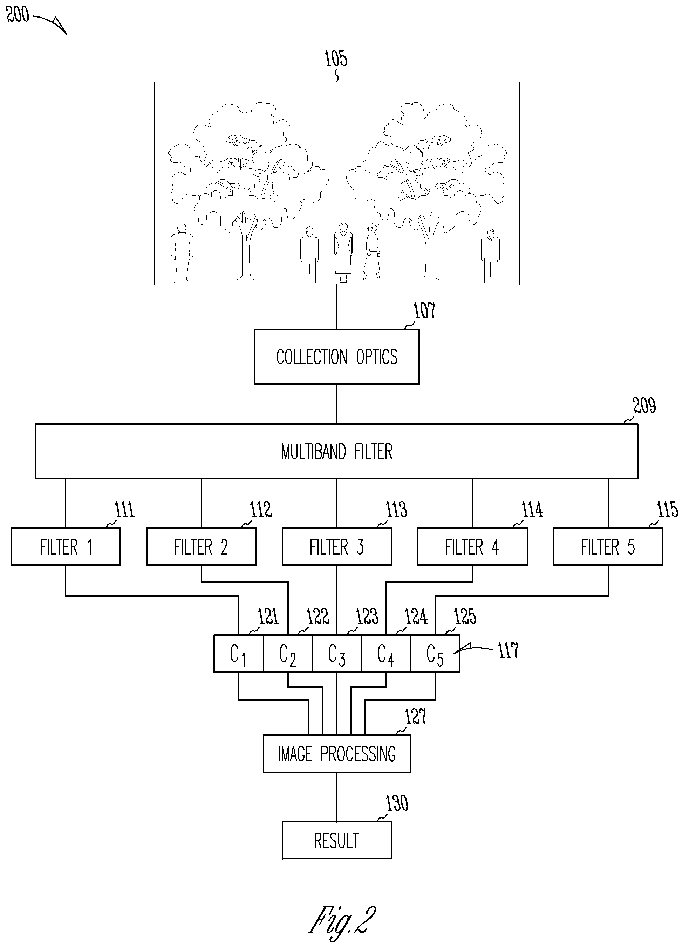

FIG. 2 is a block diagram of a multi-spectral imaging system 200 that provides remote biometric measurements in accordance with some embodiments. The multi-spectral imaging system 200 has all of the elements of the multi-spectral imaging system 100 shown in FIG. 1 and can operate in the same manner. Elements common to FIG. 1 and FIG. 2 have the same reference numerals and will not be further described herein for purposes of brevity. The multi-spectral imaging system 200 includes a multiband filter 209 that receives the light from the collection optics system 107. The multiband filter 209 may select light in several bands around several wavelengths and direct the bands of light to the narrow band filters 111-115 in accordance with some embodiments.

Some frequencies of incident light may be absorbed more by blood cells in near surface blood vessels of a human subject in the time series of images 105. The near surface blood vessels may be in the skin or the sclera of the human subject, for example. The light reflecting from the time series of images 105 varies depending on an oxygen content of blood cells in the human subject in the time series of images 105. The algorithms implemented by the image and signal processing system 127 interpret differences in the reflected light to determine the presence of one or more human subjects in the time series of images 105.

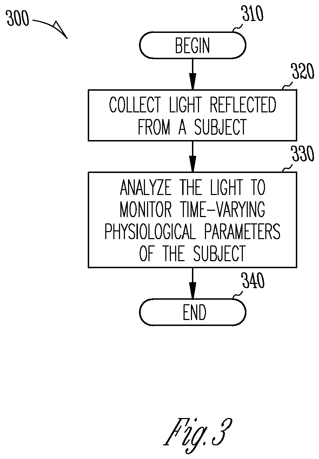

As shown in FIG. 3, still other embodiments relate to a method 300 of multi-spectral imaging for human biometric measurement. The method 300 is one embodiment of the algorithms that may be implemented by the image and signal processing system 127. The method 300 starts in box 310. As shown in box 320, the method 300 includes collecting light reflected from one or more subjects in a time series of images. The light collected may be visible light or infrared light or visible light and infrared light. The light is filtered by one or more filters around two or more wavelengths. The method 300 includes in box 330 analyzing the light to monitor time-varying physiological parameters of the subject or subjects. The frequency content of a temporal variation in the light reflected from the subject or subjects is analyzed to monitor the time-varying physiological parameters. The physiological parameters can include a heart rate, a respiration rate, blood oxygen saturation percentage and blood pressure of the subject or subjects. The method 300 ends in box 340.

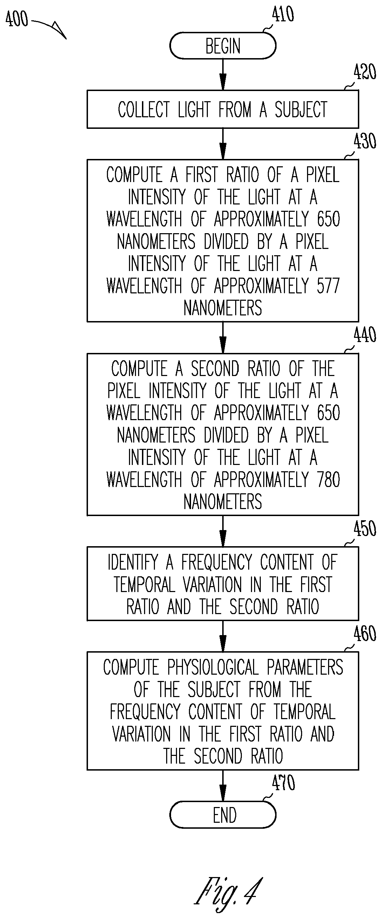

As shown in FIG. 4, still other embodiments relate to a method 400 of multi-spectral imaging for human biometric measurement. The method 400 is one embodiment of the algorithms that may be implemented by the image and signal processing system 127. The method 400 starts in box 410. As shown in box 420, the method 400 includes collecting light reflected from one or more subjects in a time series of images. The light collected may be visible light or infrared light or visible light and infrared light. The light is filtered by one or more filters around two or more wavelengths. The light may be filtered at wavelengths of approximately 547 nanometers, approximately 577 nanometers, approximately 607 nanometers, approximately 650 nanometers and approximately 780 nanometers. Reflected light may be identified in the filtered light at approximately 547 nanometers, approximately 577 nanometers and approximately 607 nanometers to indicate one or more human subjects in the time series of images 105.

As shown in box 430, the method 400 includes computing a first ratio of a pixel intensity of the light at a wavelength of approximately 650 nanometers divided by a pixel intensity of the light at a wavelength of approximately 577 nanometers. The pixel intensity is an integer from a range of integers representing the pixel between two extremes of black and white. For example, the pixel intensity may be 0 representing black or 256 representing white, or an integer between 0 and 256. As shown in box 440, the method 400 further includes computing a second ratio of the pixel intensity of the light at a wavelength of approximately 650 nanometers divided by a pixel intensity of the light at a wavelength of approximately 780 nanometers. As shown in box 450, the method 400 further includes identifying a frequency content of a temporal variation of the first ratio and the second ratio. The frequency content may be analyzed with a fast Fourier transform (FFT), a discrete Fourier transform (DFT), a continuous wavelet transform (CWT) or a discrete wavelet transform (DWT).

As shown in box 460, the method 400 further includes computing physiological parameters of the subject from the frequency content of a temporal variation of the first ratio and the second ratio. The physiological parameters can include can include a heart rate, a respiration rate, blood oxygen saturation percentage and blood pressure of the subject or subjects. The method 400 ends in box 470.

The frequency content of the temporal variation of the first ratio and the second ratio is correlated in a frequency band of approximately 0.05 to 0.5 Hertz to compute the respiration rate. The frequency content of the temporal variation of the first ratio and the second ratio is correlated in a frequency band of approximately 0.5 to 4.0 Hertz to compute the heart rate.

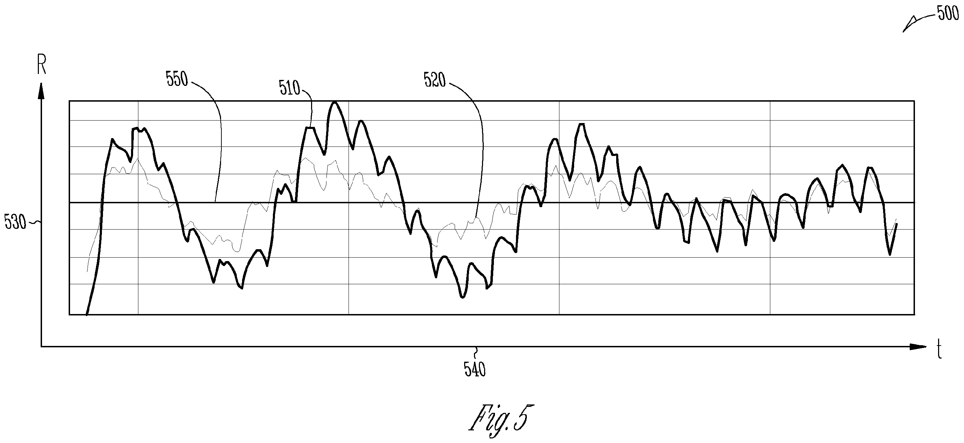

FIG. 5 is a plot 500 of a variation of the first ratio 510 and the second ratio 520 described with respect to FIG. 4 in accordance with some embodiments. A value R of the ratios is represented on a vertical axis 530 and time t is represented on a horizontal axis 540. A zero value R of the ratios is represented by a horizontal line 550.

A systolic blood pressure and a blood oxygen saturation are computed by identifying the frequency content of the temporal variation of the first ratio and the second ratio corresponding to systolic and diastolic portions of a heart beat of a human subject, and aggregating, ratioing and averaging these components. For example, time-dependent variations of the first ratio and the second ratio corresponding to the heart rate are selected by a frequency-domain band-pass filter. A mathematical operation (e.g. an inverse FFT combined with other techniques) aggregates signal values corresponding to systolic pressure and diastolic pressure of the human subject. A relative change of these signals from diastolic pressure to systolic pressure yields a measurement that is correlated with a ratio of the systolic pressure to the diastolic pressure. A temporal average of these signals enables the measurement of a relative fraction of oxygenated hemoglobin to deoxygenated hemoglobin in the blood of the human subject. An integration of the relative fraction over several heart beat intervals (or more) can determine a relative blood oxygen saturation.

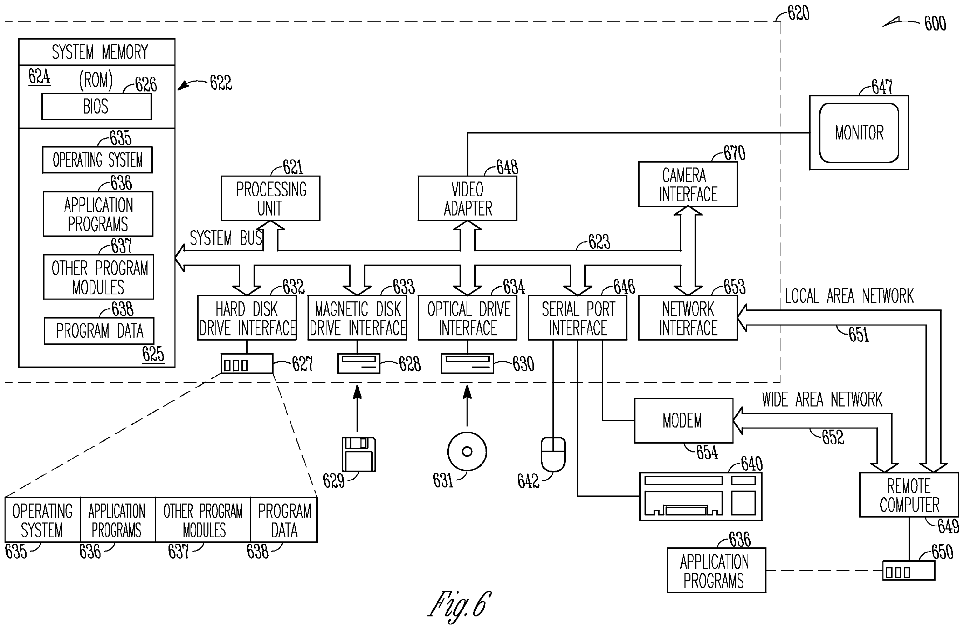

In the embodiment shown in FIG. 6, a hardware and operating environment 600 is provided that is capable of implementing any of the embodiments shown in FIGs. 3 and 4. One embodiment of the hardware and operating environment 600 includes a general purpose computing device in the form of a computer 620 (*e.g.,* a personal computer, workstation, or server), including one or more processing units 621, a system memory 622, and a system bus 623 that operatively couples various system components including the system memory 622 to the processing unit 621. There may be only one or there may be more than one processing unit 621, such that the processor of computer 620 comprises a single central-processing unit (CPU), or a plurality of processing units, commonly referred to as a multiprocessor or parallel-processor environment. A multiprocessor system can include cloud-computing environments. In various embodiments, computer 620 is a conventional computer, a distributed computer, or any other type of computer.

The system bus 623 may be any of several types of bus structures including a memory bus or memory controller, a peripheral bus, and a local bus using any of a variety of bus architectures. The system memory can also be referred to as simply the memory, and, in some embodiments, includes read-only memory (ROM) 624 and random-access memory (RAM) 625. A basic input/output system (BIOS) program 626, containing the basic routines that help to transfer information between elements within the computer 620, such as during start-up, may be stored in ROM 624. The computer 620 further includes a hard disk drive 627 for reading from and writing to a hard disk, not shown, a magnetic disk drive 628 for reading from or writing to a removable magnetic disk 629, and an optical disk drive 630 for reading from or writing to a removable optical disk 631 such as a CD ROM or other optical media. The hard disk drive 627, magnetic disk drive 628, and optical disk drive 630 couple with a hard disk drive interface 632, a magnetic disk drive interface 633, and an optical disk drive interface 634, respectively. The drives and their associated computer-readable media provide non-volatile storage of computer-readable instructions, data structures, program modules and other data for the computer 620.

A plurality of program modules may be stored on the hard disk, magnetic disk 629, optical disk 631, ROM 624, or RAM 625, including an operating system 635, one or more application programs 636, other program modules 637, and program data 638. A plug in containing a security transmission engine for the present invention may be resident on any one or number of these computer-readable media.

A user may enter commands and information into computer 620 through input devices such as a keyboard 640 and pointing device 642. Other input devices (not shown) can include a microphone, joystick, game pad, satellite dish, scanner, or the like. These other input devices are often connected to the processing unit 621 through a serial port interface 646 that is coupled to the system bus 623, but may be connected by other interfaces, such as a parallel port, game port, or a universal serial bus (USB). A monitor 647 or other type of display device can also be connected to the system bus 623 via an interface, such as a video adapter 648. The monitor 647 can display a graphical user interface for the user. In addition to the monitor 647, computers can include other peripheral output devices (not shown), such as speakers and printers.

The computer 620 may operate in a networked environment using logical connections to one or more remote computers or servers, such as remote computer 649. These logical connections are achieved by a communication device coupled to or a part of the computer 620; the invention is not limited to a particular type of communications device. The remote computer 649 may be another computer, a server, a router, a network PC, a client, a peer device or other common network node, and can include many or all of the elements described above I/O relative to the computer 620, although only a memory storage device 650 has been illustrated. The logical connections depicted in FIG. 6 include a local area network (LAN) 651 and/or a wide area network (WAN) 652. Such networking environments are commonplace in office networks, enterprise-wide computer networks, intranets and the internet, which are all types of networks.

The computer 620 is connected to the LAN 651 through a network interface or adapter 653. In some embodiments, when used in a WAN-networking environment, the computer 620 can include a modem 654 or any other type of communications device, such as a wireless transceiver, for establishing communications over the wide-area network 652, such as the internet. The modem 654, which may be internal or external, is connected to the system bus 623 via the serial port interface 646. In a networked environment, program modules depicted relative to the computer 620 may be stored in the remote memory storage device 650 of remote computer, or server 649.

The computer 620 may be connected to the image capture zones 121-125 in the multi-spectral imaging system 100 through a camera interface or adapter 670 to receive the digital images generated by the image capture zones 121-125. The computer 620 can implement algorithms to produce the results 130 including temporal signatures of physiological parameters of subjects present in the time series of images 105.

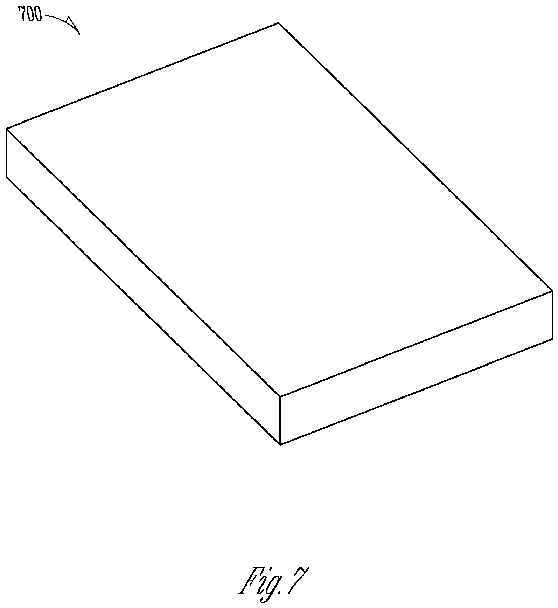

FIG. 7 is a block diagram of an integrated circuit chip 700 in accordance with some embodiments. The integrated circuit chip 700 includes hardware such as a state machine, an application-specific integrated circuit (ASIC) or a field-programmable gate array (FPGA) that is capable of implementing any of the embodiments shown in FIGs. 3 and 4.

The following claims are hereby incorporated into the detailed description, with each claim standing on its own as a separate embodiment.

## Claims

1. A method implemented by an image and signal processing system (127), the method comprising:
collecting light (320; 420) reflected from a subject as digital images; and
analyzing (330; 430-460) the light to monitor time-varying physiological parameters of the subject for biometric characterization of one or more human physiological parameters of the subject;
wherein analyzing the light further comprises identifying the subject as a human subject from light reflected from the subject at first, second and third wavelengths,
wherein the first wavelength comprises a wavelength in the range of 547 nanometers +/- 20 nanometers,
wherein the second wavelength comprises a wavelength in the range of 577 nanometers +/- 20 nanometers, and
wherein the third wavelength comprises a wavelength in the range of 607 nanometers +/- 20 nanometers;
wherein analyzing the light further comprises:
computing (430) a first ratio (516) of a pixel intensity of light reflected from the subject at a fourth wavelength divided by a pixel intensity of light reflected from the subject at the second wavelength;
computing (440) a second ratio (520) of the pixel intensity of the light reflected from the subject at the fourth wavelength divided by a pixel intensity of the light reflected from the subject at a fifth wavelength;
identifying (450) a frequency content of a temporal variation of the first ratio and the second ratio; and
computing (460) a heart rate of the human subject from the frequency content of a temporal variation of the first ratio and the second ratio in a frequency band of 0.5 to 4.0 Hertz and computing (460) a respiration rate of the human subject from the frequency content of a temporal variation of the first ratio and the second ratio in a frequency band of 0.05 to 0.5 Hertz,
wherein the fourth wavelength comprises a wavelength in the range of 650 nanometers +/- 20 nanometers, and
wherein the fifth wavelength comprises a wavelength in the range of 780 nanometers +/- 20 nanometers.

2. The method of claim 1, wherein collecting (320; 420) the light further comprises collecting the light reflected from the subject remotely.

3. The method of claim 1, wherein:
collecting (320; 420) the light reflected from the subject further comprises collecting light reflected from a plurality of subjects; and
analyzing (330; 430-460) the light further comprises analyzing the light to monitor time-varying physiological parameters of each subject.

4. The method of claim 1, wherein the physiological parameters further comprise one or more of blood oxygen saturation percentage and blood pressure.

5. The method of claim 1, wherein:
collecting (320; 420) the light further comprises collecting light having a response to the subject; and
analyzing (330; 430-460) the light further comprises detecting differences in the collected light.

6. The method of claim 1, wherein analyzing the light further comprises:
identifying a frequency content of the temporal variation of the light; and
computing physiological parameters of the subject from the frequency content of the temporal variation of the light.

7. The method of claim 1, wherein collecting (320; 420) the light further comprises collecting light reflected from the subject comprising visible light or infrared light or visible light and infrared light.

8. A system (100; 200) comprising:
collection optics (107) to receive light reflected from a subject;
a plurality of filters (111-115; 209) to filter the light at first, second, third, fourth and fifth wavelengths;
a plurality of image capture zones (121-125), each image capture zone to receive filtered light from the filters and to generate a digital image to represent the filtered light; and
an image and signal processing system (127) coupled to the image capture zones to receive the digital images generated by the image capture zones to
identify the subject as a human subject from the digital images from the light at the first, second and third wavelengths; and
compute a heart rate and a respiration rate of the human subject indicated by the digital images from the light at the second wavelength, a fourth wavelength and a fifth wavelength;
wherein the first wavelength comprises a wavelength in the range of 547 nanometers +/- 20 nanometers,
wherein the second wavelength comprises a wavelength in the range of 577 nanometers +/- 20 nanometers,
wherein the third wavelength comprises a wavelength in the range of 607 nanometers +/- 20 nanometers,
wherein the fourth wavelength comprises a wavelength in the range of 650 nanometers +/- 20 nanometers, and
wherein the fifth wavelength comprises a wavelength in the range of 780 nanometers +/- 20 nanometers;
wherein the image and signal processing system (127) is further configured to:
compute a first ratio of a pixel intensity of the light at the fourth wavelength divided by a pixel intensity of the light at the second wavelength;
compute a second ratio of the pixel intensity of the light at the fourth wavelength divided by a pixel intensity of the light at the fifth wavelength;
compute the heart rate of the human subject from a frequency content of a temporal variation of the first ratio and the second ratio in a frequency band of 0.5 to 4.0 Hertz and compute a respiration rate of the human subject from the frequency content of a temporal variation of the first ratio and the second ratio in a frequency band of 0.05 to 0.5 Hertz.

9. The system of claim 8, wherein the image and signal processing system (127) is further structured to compute a blood oxygen saturation percentage and a blood pressure of the human subject from a frequency content of the digital images generated by the image capture zones.

10. The system of claim 9, wherein the filters (111-115; 209) comprise:
a first filter (209) to filter the light; and
a plurality of narrow band filters (111-115), each narrow band filter to filter the light from the first filter around one of the wavelengths.

11. The system of claim 8, wherein the image and signal processing system (127) is structured to receive the digital images generated by the image capture zones (121-125) for a time series of images of the subject.

12. The system of claim 8, wherein each image capture zone (121-125) comprises a Multi-Spectral Imaging sensor.

## Patentansprüche

1. Verfahren, das durch ein Bild- und Signalverarbeitungssystem (127) implementiert wird, wobei das Verfahren umfasst:
Sammeln von Licht (320; 420), das von einem Subjekt reflektiert wird, als digitale Bilder; und
Analysieren (330; 430-460) des Lichts, um zeitlich veränderliche physiologische Parameter des Subjekts für eine biometrische Charakterisierung von einem oder mehreren humanen physiologischen Parametern des Subjekts zu überwachen;
wobei Analysieren des Lichts weiter umfasst Identifizieren des Subjekts als ein humanes Subjekt aus Licht, das von dem Subjekt mit ersten, zweiten und dritten Wellenlängen reflektiert wird,
wobei die erste Wellenlänge eine Wellenlänge in dem Bereich von 547 Nanometer +/- 20 Nanometer umfasst,
wobei die zweite Wellenlänge eine Wellenlänge in dem Bereich von 577 Nanometer +/- 20 Nanometer umfasst und
wobei die dritte Wellenlänge eine Wellenlänge in dem Bereich von 607 Nanometer +/- 20 Nanometer umfasst;
wobei Analysieren des Lichts weiter umfasst:
Berechnen (430) eines ersten Verhältnisses (516) von einer Pixelintensität von Licht, das von dem Subjekt mit einer vierten Wellenlänge reflektiert wird, geteilt durch eine Pixelintensität von Licht, das von dem Subjekt mit der zweiten Wellenlänge reflektiert wird;
Berechnen (440) eines zweiten Verhältnisses (520) von der Pixelintensität von dem Licht, das von dem Subjekt mit der vierten Wellenlänge reflektiert wird, geteilt durch eine Pixelintensität von dem Licht, das von dem Subjekt mit einer fünften Wellenlänge reflektiert wird;
Identifizieren (450) eines Frequenzinhalts von einer zeitlichen Variation von dem ersten Verhältnis und dem zweiten Verhältnis; und
Berechnen (460) einer Herzschlagrate des humanen Subjekts aus dem Frequenzinhalt von einer zeitlichen Variation von dem ersten Verhältnis und dem zweiten Verhältnis in einem Frequenzband von 0,5 bis 4,0 Hertz und Berechnen (460) einer Atmungsrate des humanen Subjekts aus dem Frequenzinhalts von einer zeitlichen Variation von dem ersten Verhältnis und dem zweiten Verhältnis in einem Frequenzband von 0,05 bis 0,5 Hertz,
wobei die vierte Wellenlänge eine Wellenlänge in dem Bereich von 650 Nanometer +/- 20 Nanometer umfasst und
wobei die fünfte Wellenlänge eine Wellenlänge in dem Bereich von 780 Nanometer +/- 20 Nanometer umfasst.

2. Verfahren nach Anspruch 1, wobei Sammeln (320; 420) des Lichts weiter umfasst entfernt Sammeln des Lichts, das von dem Subjekt reflektiert wird.

3. Verfahren nach Anspruch 1, wobei:
Sammeln (320; 420) des Lichts, das von dem Subjekt reflektiert wird, weiter umfasst Sammeln von Licht, das von einer Vielzahl von Subjekten reflektiert wird; und
Analysieren (330; 340-460) des Lichts weiter umfasst Analysieren des Lichts, um zeitlich veränderliche physiologische Parameter von jedem Subjekt zu überwachen.

4. Verfahren nach Anspruch 1, wobei die physiologischen Parameter weiter eines oder mehrere von Blutsauerstoffanteil und Blutdruck umfassen.

5. Verfahren nach Anspruch 1, wobei:
Sammeln (320; 420) des Lichts weiter umfasst Sammeln von Licht mit einer Antwort auf das Subjekt; und
Analysieren (330; 430-460) des Lichts weiter umfasst Detektieren von Unterschieden in dem gesammelten Licht.

6. Verfahren nach Anspruch 1, wobei Analysieren des Lichts weiter umfasst:
Identifizieren eines Frequenzinhalts von der zeitlichen Variation des Lichts; und
Berechnen von physiologischen Parametern des Subjekts aus dem Frequenzinhalts von der zeitlichen Variation des Lichts.

7. Verfahren nach Anspruch 1, wobei Sammeln (320; 420) des Lichts weiter umfasst Sammeln von Licht, das von dem Subjekt reflektiert wird, umfassend sichtbares Licht oder Infrarotlicht oder sichtbares Licht und Infrarotlicht.

8. System (100; 200) umfassend:
eine Sammeloptik (107) zum Empfangen von Licht, das von einem Subjekt reflektiert wird;
eine Vielzahl von Filtern (111-115; 209) zum Filtern des Lichts mit ersten, zweiten, dritten, vierten und fünften Wellenlängen;
eine Vielzahl von Bilderfassungszonen (121-125), wobei jede Bilderfassungszone zum Empfangen von gefiltertem Licht von den Filtern und zum Erzeugen eines digitalen Bildes, um das gefilterte Licht darzustellen, ist; und
ein Bild- und Signalverarbeitungssystem (127), das mit den Bilderfassungszonen gekoppelt ist, zum Empfangen der digitalen Bilder, die durch die Bilderfassungszonen erzeugt werden, zum
Identifizieren des Subjekts als ein humanes Subjekt aus den digitalen Bildern von dem Licht mit den ersten, zweiten und dritten Wellenlängen; und
Berechnen einer Herzschlagrate und einer Atmungsrate des humanen Subjekts, angezeigt durch die digitalen Bilder von dem Licht mit der zweiten Wellenlänge, einer vierten Wellenlänge und einer fünften Wellenlänge;
wobei die erste Wellenlänge eine Wellenlänge in dem Bereich von 547 Nanometer +/- 20 Nanometer umfasst,
wobei die zweite Wellenlänge eine Wellenlänge in dem Bereich von 577 Nanometer +/- 20 Nanometer umfasst,
wobei die dritte Wellenlänge eine Wellenlänge in dem Bereich von 607 Nanometer +/- 20 Nanometer umfasst,
wobei die vierte Wellenlänge eine Wellenlänge in dem Bereich von 650 Nanometer +/- 20 Nanometer umfasst und
wobei die fünfte Wellenlänge eine Wellenlänge in dem Bereich von 780 Nanometer +/- 20 Nanometer umfasst;
wobei das Bild- und Signalverarbeitungssystem (127) weiter konfiguriert ist zum:
Berechnen eines ersten Verhältnisses von einer Pixelintensität von dem Licht mit der vierten Wellenlänge geteilt durch eine Pixelintensität von dem Licht mit der zweiten Wellenlänge;
Berechnen eines zweiten Verhältnisses von der Pixelintensität von dem Licht mit der vierten Wellenlänge geteilt durch eine Pixelintensität von dem Licht mit der fünften Wellenlänge;
Berechnen der Herzschlagrate des humanen Subjekts aus dem Frequenzinhalt von einer zeitlichen Variation von dem ersten Verhältnis und dem zweiten Verhältnis in einem Frequenzband von 0,5 bis 4,0 Hertz und Berechnen einer Atmungsrate des humanen Subjekts aus dem Frequenzinhalts von einer zeitlichen Variation von dem ersten Verhältnis und dem zweiten Verhältnis in einem Frequenzband von 0,05 bis 0,5 Hertz.

9. System nach Anspruch 8, wobei das Bild- und Signalverarbeitungssystem (127) weiter strukturiert ist zum Berechnen eines Blutsauerstoffsättigungsanteils und eines Blutdrucks des humanen Subjekts aus einem Frequenzinhalt der digitalen Bilder, die durch die Bilderfassungszonen erzeugt werden.

10. System nach Anspruch 9, wobei die Filter (111-115; 209) umfassen:
einen ersten Filter (209) zum Filtern des Lichts; und
eine Vielzahl von Schmalbandfiltern (111-115), wobei jeder Schmalbandfilter zum Filtern des Lichts von dem ersten Filter um eine der Wellenlängen ist.

11. System nach Anspruch 8, wobei das Bild- und Signalverarbeitungssystem (127) strukturiert ist zum Empfangen der digitalen Bilder, die durch die Bilderfassungszonen (121-125) erzeugt werden, für eine Zeitfolge von Bildern des Subjekts.

12. System nach Anspruch 8, wobei jede Bilderfassungszone (121-125) einen multispektralen Bildgebungssensor umfasst.

## Revendications

1. Procédé mise en oeuvre par un système de traitement d'image et de signal (127), le procédé comprenant :
le recueil de la lumière (320 ; 420) réfléchie par un sujet sous forme d'images numériques ; et
l'analyse (330 ; 430-460) de la lumière pour surveiller des paramètres physiologiques variables dans le temps du sujet pour une caractérisation biométrique d'un ou plusieurs paramètres physiologiques humains du sujet ;
dans lequel l'analyse de la lumière comprend en outre l'identification du sujet en tant que sujet humain à partir de la lumière réfléchie par le sujet à des première, deuxième et troisième longueurs d'onde,
dans lequel la première longueur d'onde comprend une longueur d'onde dans la plage de 547 nanomètres +/- 20 nanomètres,
dans lequel la deuxième longueur d'onde comprend une longueur d'onde dans la plage de 577 nanomètres +/- 20 nanomètres, et
dans lequel la troisième longueur d'onde comprend une longueur d'onde dans la plage de 607 nanomètres +/- 20 nanomètres ;
dans lequel l'analyse de la lumière comprend en outre :
le calcul (430) d'un premier rapport (516) d'une intensité de pixel de la lumière réfléchie par le sujet à une quatrième longueur d'onde divisée par une intensité de pixel de la lumière réfléchie par le sujet à la deuxième longueur d'onde ;
le calcul (440) d'un deuxième rapport (520) de l'intensité de pixel de la lumière réfléchie par le sujet à la quatrième longueur d'onde divisée par une intensité de pixel de la lumière réfléchie par le sujet à une cinquième longueur d'onde ;
l'identification (450) d'un contenu fréquentiel d'une variation temporelle du premier rapport et du deuxième rapport ; et
le calcul (460) d'un rythme cardiaque du sujet humain à partir du contenu fréquentiel d'une variation temporelle du premier rapport et du deuxième rapport dans une bande de fréquence de 0,5 à 4,0 Hertz et le calcul (460) d'un rythme respiratoire du sujet humain à partir du contenu fréquentiel d'une variation temporelle du premier rapport et du deuxième rapport dans une bande de fréquence de 0,05 à 0,5 Hertz,
dans lequel la quatrième longueur d'onde comprend une longueur d'onde dans la plage de 650 nanomètres +/- 20 nanomètres, et
dans lequel la cinquième longueur d'onde comprend une longueur d'onde dans la plage de 780 nanomètres +/- 20 nanomètres.

2. Procédé selon la revendication 1, dans lequel le recueil (320 ; 420) de la lumière comprend en outre le recueil de la lumière réfléchie par le sujet, à distance.

3. Procédé selon la revendication 1, dans lequel :
le recueil (320 ; 420) de la lumière réfléchie par le sujet comprend en outre le recueil de la lumière réfléchie par une pluralité de sujets ; et
l'analyse (330 ; 430-460) de la lumière comprend en outre l'analyse de la lumière pour surveiller des paramètres physiologiques variables dans le temps de chaque sujet.

4. Procédé selon la revendication 1, dans lequel les paramètres physiologiques comprennent en outre un ou plusieurs parmi le pourcentage de saturation en oxygène du sang et la pression sanguine.

5. Procédé selon la revendication 1, dans lequel :
le recueil (320 ; 420) de la lumière comprend en outre le recueil de la lumière présentant une réponse au sujet ; et
l'analyse (330 ; 430-460) de la lumière comprend en outre la détection de différences dans la lumière recueillie.

6. Procédé selon la revendication 1, dans lequel l'analyse de la lumière comprend en outre :
l'identification d'un contenu fréquentiel de la variation temporelle de la lumière ; et
le calcul de paramètres physiologiques du sujet à partir du contenu fréquentiel de la variation temporelle de la lumière.

7. Procédé selon la revendication 1, dans lequel le recueil (320 ; 420) de la lumière comprend en outre le recueil de la lumière réfléchie par le sujet comprenant de la lumière visible ou de la lumière infrarouge ou de la lumière visible et de la lumière infrarouge.

8. Système (100 ; 200) comprenant :
des optiques de recueil (107) pour recevoir la lumière réfléchie par un sujet ;
une pluralité de filtres (111-115 ; 209) pour filtrer la lumière en tant que première, deuxième, troisième, quatrième et cinquième longueurs d'onde ;
une pluralité de zones de capture d'image (121-125), chaque zone de capture d'image permettant de recevoir la lumière filtrée provenant des filtres et de générer une image numérique pour représenter la lumière filtrée ; et
un système de traitement d'image et de signal (127) couplé aux zones de capture d'image pour recevoir les images numériques générées par les zones de capture d'image pour
identifier le sujet en tant que sujet humain à partir des images numériques de la lumière aux première, deuxième et troisième longueurs d'onde ; et
calculer un rythme cardiaque et un rythme respiratoire du sujet humain, indiqués par les images numériques de la lumière à la deuxième longueur d'onde, à une quatrième longueur d'onde et à une cinquième longueur d'onde ;
dans lequel la première longueur d'onde comprend une longueur d'onde dans la plage de 547 nanomètres +/- 20 nanomètres,
dans lequel la deuxième longueur d'onde comprend une longueur d'onde dans la plage de 577 nanomètres +/- 20 nanomètres,
dans lequel la troisième longueur d'onde comprend une longueur d'onde dans la plage de 607 nanomètres +/- 20 nanomètres,
dans lequel la quatrième longueur d'onde comprend une longueur d'onde dans la plage de 650 nanomètres +/- 20 nanomètres, et
dans lequel la cinquième longueur d'onde comprend une longueur d'onde dans la plage de 780 nanomètres +/- 20 nanomètres ;
dans lequel le système de traitement d'image et de signal (127) est en outre configuré pour :
calculer un premier rapport d'une intensité de pixel de la lumière à la quatrième longueur d'onde divisée par une intensité de pixel de la lumière à la deuxième longueur d'onde ;
calculer un deuxième rapport de l'intensité de pixel de la lumière à la quatrième longueur d'onde divisée par une intensité de pixel de la lumière à la cinquième longueur d'onde ;
calculer un rythme cardiaque du sujet humain à partir du contenu fréquentiel d'une variation temporelle du premier rapport et du deuxième rapport dans une bande de fréquence de 0,5 à 4,0 Hertz et calculer un rythme respiratoire du sujet humain à partir du contenu fréquentiel d'une variation temporelle du premier rapport et du deuxième rapport dans une bande de fréquence de 0,05 à 0,5 Hertz,

9. Système selon la revendication 8, dans lequel le système de traitement d'image et de signal (127) est en outre structuré pour calculer un pourcentage de saturation en oxygène du sang et une pression sanguine du sujet humain à partir d'un contenu fréquentiel des images numériques générées par les zones de capture d'image.

10. Système selon la revendication 9, dans lequel les filtres (111-115 ; 209) comprennent :
un premier filtre (209) pour filtrer la lumière ; et
une pluralité de filtres à bande étroite (111-115), chaque filtre à bande étroite permettant de filtrer la lumière provenant du premier filtre autour d'une des longueurs d'onde.

11. Système selon la revendication 8, dans lequel le système de traitement d'image et de signal (127) est structuré pour recevoir les images numériques générées par les zones de capture d'image (121-125) pour une série temporelle d'images du sujet.

12. Système selon la revendication 8, dans lequel chaque zone de capture d'image (121-125) comprend un capteur d'imagerie multispectrale.
